# FASCICULE DE BREVET EUROPEEN

(11) **EP 1 646 651 B1**
(45) Date de publication et mention de la délivrance du brevet: **28.11.2012**
(21) Numéro de dépôt: 04767706.7
(22) Date de dépôt: 16.07.2004
(51) Int. Cl.: C07K 14/66, C07K 7/06, A61K 38/04

(54) **NOUVEAUX CONJUGUES PEPTIDIQUES UTILES DANS LE TRAITEMENT PREVENTIF ET CURATIF DE L'ALOPECIE**
NEUE PEPTIDISCHE KONJUGATE FÜR EINE ALOPECIE VERHINDERNDE UND HEILENDE BEHANDLUNG
NOVEL PEPTIDIC CONJUGATES FOR ALOPECIA PREVENTIVE AND CURATIVE TREATMENT

(30) Priorité: 18.07.2003 FR 0308797
(43) Date de publication de la demande: 19.04.2006
(73) Titulaire: INSTITUT EUROPEEN DE BIOLOGIE CELLULAIRE, 31520 Ramonville Saint-Agne (FR)
(72) Inventeur: PINEL, Anne-Marie, F-31400 Toulouse (FR); HOCQUAUX, Michel, F-75012 Paris (FR)
(74) Mandataire: Faivre Petit, Frédérique
(86) Numéro de dépôt international: PCT/FR2004/001882
(87) Numéro de publication internationale: WO 2005/010027

(56) Documents cités:
- WO-A-97/18239
- DE-A- 19 608 229
- TOSTI A ET AL: "THYMOPENTIN IN THE TREATMENT OF SEVERE ALOPECIA AREATA" DERMATOLOGICA (BASEL), vol. 177, no. 3, 1988, pages 170-174, XP009022230 ISSN: 0011-9075

### LISTE DE SEQUENCES

<110> INSTITUT EUROPEEN DE BIOLOGIE CELLULAIRE
<120> NOUVEAUX CONJUGUES PEPTIDIQUES UTILES DANS LE TRAITEMENT PREVENTIF ET CURATIF DE L'ALOPECIE
<130> D21279
<150> FR 03/08 797
   <151> 2003-07-18
<160> 11
<170> PatentIn version 3.2
<210> 1
   <211> 5
   <212> PRT
   <213> Séquence artificielle
<220>
   <223> Peptide
<220>
   <221> misc_feature
   <222> (1) .. (1)
   <223> Xaa peut être les séquences Glu-Gln-Arg, Arg-Lys, Arg-Lys-Asp ou l'acide aminé Arg ou une liaison
<220>
   <221> misc_feature
   <222> (5)..(5)
   <223> Xaa Peut être les séquences Tyr-Val-Gln-Leu-Tyr-Amidée, Leu-DOPA, les acides aminés Dopa amidé ou HomoPhe amidé
<400> 1
   Xaa Lys Asp Val Xaa
   1               5
<210> 2
   <211> 5
   <212> PRT
   <213> Séquence artificielle
<220>
   <223> Peptide
<220>
   <221> misc_feature
   <222> (1)..(1)
   <223> Xaa Peut être les séquences Gly-Gln-Gln ou Glu-Gln
<220>
   <221> misc_feature
   <222> (5)..(5)
   <223> Xaa Peut être les séquences Tyr-Val-Gln-Leu-Tyr-Amidée, Leu-DOPA, Val-Tyr, Val-Tyr-amidé, ou les acides aminés Tyr, Tyr amidé, Dopa amidé ou HomoPhe amidé
<400> 2
   Xaa Lys Asp Val Xaa
   1               5
<210> 3
   <211> 5
   <212> PRT
   <213> Séquence artificielle
<220>
   <223> Peptide
<220>
   <221> MOD_RES
   <222> (1) .. (1)
   <223> BLOCKED
<220>
   <221> misc_feature
   <222> (1)..(1)
   <223> Xaa peut être les séquences Glu-Gln-Arg, Arg-Lys, Arg-Lys-Asp ou l'acide aminé Arg ou une liaison
<220>
   <221> misc_feature
   <222> (5) .. (5)
   <223> Xaa Peut être les séquences Tyr-Val-Gln-Leu-Tyr-Amidée, Leu-DOPA, les acides aminés Dopa amidé ou HomoPhe amidé
<400> 3
   Xaa Lys Asp Val Xaa
   1               5
<210> 4
   <211> 5
   <212> PRT
   <213> Séquence artificielle
<220>
   <223> Peptide
<220>
   <221> MOD_RES
   <222> (1).. (1)
   <223> BLOCKED
<220>
   <221> misc_feature
   <222> (1)..(1)
   <223> Xaa Peut être les séquences Gly-Gln-Gln ou Glu-Gln
<220>
   <221> misc_feature
   <222> (5) .. (5)
   <223> Xaa Peut être les séquences Tyr-Val-Gln-Leu-Tyr-Amidée, Leu-DOPA, Val-Tyr, Val-Tyr-amidé, ou les acides aminés Tyr, Tyr amidé, Dopa amidé ou HomoPhe amidé
<400> 4
   Xaa Lys Asp Val Xaa
   1               5
<210> 5
   <211> 5
   <212> PRT
   <213> Séquence artificielle
<220>
   <223> Peptide
<220>
   <221> MOD_RES
   <222> (1)..(1)
   <223> BLOCKED
<220>
   <221> MOD_RES
   <222> (5)..(5)
   <223> AMIDATION
<220>
   <221> misc_feature
   <222> (5)..(5)
   <223> Xaa = homophenylalanine amidée.
<400> 5
   Arg Lys Asp Val Xaa
   1               5
<210> 6
   <211> 4
   <212> PRT
   <213> Séquence artificielle
<220>
   <223> Peptide
<220>
   <221> MOD_RES
   <222> (1) .. (1)
   <223> BLOCKED
<220>
   <221> MOD_RES
   <222> (4)..(4)
   <223> AMIDATION
<220>
   <221> misc_feature
   <222> (4) .. (4)
   <223> Xaa = dihydrophenylalanine amidée.
<400> 6
   Lys Asp Val Xaa
   1
<210> 7
   <211> 5
   <212> PRT
   <213> Séquence artificielle
<220>
   <223> Peptide
<220>
   <221> MOD_RES
   <222> (1) .. (1)
   <223> BLOCKED
<220>
   <221> MOD_RES
   <222> (5) .. (5)
   <223> AMIDATION
<220>
   <221> misc_feature
   <222> (5) .. (5)
   <223> Xaa = dihydrophenylalaline amidée.
<400> 7
   Arg Lys Asp Val Xaa
   1               5
<210> 8
   <211> 4
   <212> PRT
   <213> Séquence artificielle
<220>
   <223> Peptide
<220>
   <221> misc_feature
   <222> (1) .. (1)
   <223> Xaa peut être 1 à trois résidus Lys ou MeLys
<400> 8
   Xaa Gly His Lys
   1
<210> 9
   <211> 4
   <212> PRT
   <213> Séquence artificielle
<220>
   <223> Peptide
<220>
   <221> misc_feature
   <222> (1)..(1)
   <223> Xaa peut être 1 à trois résidus Lys ou MeLys
<220>
   <221> MOD_RES
   <222> (4)..(4)
   <223> AMIDATION
<400> 9
   Xaa Gly His Lys
   1
<210> 10
   <211> 4
   <212> PRT
   <213> Séquence artificielle
<220>
   <223> Peptide
<220>
   <221> MOD_RES
   <222> (1)..(1)
   <223> BLOCKED
<220>
   <221> misc_feature
   <222> (1)..(1)
   <223> Xaa peut être 1 à trois résidus Lys, MeLys ou une liaison.
<400> 10
   Xaa Gly His Lys
   1
<210> 11
   <211> 4
   <212> PRT
   <213> Séquence artificielle
<220>
   <223> Peptide
<220>
   <221> MOD_RES
   <222> (1)..(1)
   <223> BLOCKED
<220>
   <221> misc_feature
   <222> (1)..(1)
   <223> Xaa peut être 1 à trois résidus Lys, MeLys ou une liaison.
<220>
   <221> MOD_RES
   <222> (4) .. (4)
   <223> AMIDATION
<400> 11
   Xaa Gly His Lys
   1

## Description

La présente invention a pour objet de nouveaux conjugués peptidiques contenant la séquence Lys-Asp-Val, utiles en dermatologie ou cosmétologie, notamment pour stimuler la croissance des cheveux ou freiner leur chute.

Durant toute la vie d'un individu, la croissance des cheveux et leur renouvellement sont déterminés par l'activité des follicules pileux. Ils effectuent un cycle régulier composé de trois phases : anagène, catagène et télogène, qui sont chacune caractérisées par des mécanismes moléculaires et cellulaires bien précis :
- Durant la phase anagène qui dure environ trois ans, les cellules de la papille dermique "envoient" des signaux aux cellules souches présentes dans le bulbe. Les cellules compétentes qui reçoivent ces signaux migrent alors vers la matrice du follicule pileux, on parle alors de cellules matricielles. Dans cette zone, les cellules de la papille dermique émettent des signaux supplémentaires qui permettent aux cellules matricielles de proliférer dans un premier temps puis de se différencier ce qui permet l'allongement de la tige pilaire. Lors de cette phase, le follicule pileux migre au travers du derme pour se retrouver en anagène VI ancré dans l'hypoderme au contact du tissu adipeux.
- La phase qui suit, dite catagène, est une phase courte qui dure environ trois semaines durant lesquelles les cellules de la partie inférieure du follicule pileux rentrent en apoptose permettant ainsi la dégénerescence du follicule pileux.
- La phase restante, dite télogène, est une phase de latence caractérisée par l'inactivité du follicule pileux durant trois mois et la chute du cheveu avant une nouvelle entrée en phase anagène.

L'apparence étant à notre époque un facteur social primordial, la perte de cheveux est un réel problème qui peut être vécu comme un handicap social par certaines personnes. Chez l'homme, il s'agit dans la plupart des cas d'alopécie androgénique. Ce type d'alopécie est donc dû à un défaut de catabolisme des androgènes et plus précisément de la testostérone au niveau du follicule pileux par les cellules de papille dermique. En effet, il y a accumulation d'un métabolite de la testostérone, la DHT (métabolite qui est produit par action de la 5α - réductase sur la testostérone), au niveau des follicules pileux. Dans un processus normal, ce composé est dégradé puis éliminé dans les urines. A l'heure actuelle, les inhibiteurs de 5α -réductase sont utilisés dans ce type d'alopécie pour ralentir la chute des cheveux.

L'ensemble des connaissances actuelles concernant la biologie du cheveu et du cuir chevelu, les alopécies et les affections du cuir chevelu, leurs traitements sont rassemblés dans : "Pathologie du cheveu et du cuir chevelu" P. Bouhanna et P. Reygagne - Editions Masson.

On recherche depuis de nombreuses années, dans l'industrie cosmétique ou pharmaceutique, des substances permettant de supprimer ou de réduire l'effet de l'alopécie et notamment d'induire ou de stimuler la croissance des cheveux ou de diminuer leur chute.

Un certain nombre de composés sont déjà utilisés comme le minoxidil, le finastéride.

Certains peptides sont connus pour leur action stimulante de la pousse du cheveu toutefois aucun document ne divulgue des peptides ou conjugués peptidiques objets de la présente invention.

La demanderesse a synthétisé de nouveaux peptides et conjugués peptidiques contenant la séquence Lys-Asp-Val capables de lutter contre l'alopécie.

La présente invention a donc pour objet un peptide répondant à la formule (I)

W-Lys-Asp-Val-Z (I)

(SEQ ID N°1-2)
ou son conjugué peptidique répondant à la formule (II)

A-W-Lys-Asp-Val-Z (II)

(SEQ ID N°3-4)
dans lesquelles
A représente le radical correspondant à
- un acide monocarboxylique de formule générale (III)

   HOOC-R (III)

   dans laquelle
   R représente un radical aliphatique en C1-C24, linéaire ou ramifié, éventuellement substitué par un groupe hydroxy, pouvant comporter une ou plusieurs insaturations, avantageusement de 1 à 6 insaturations,
- l'acide lipoïque ou sa forme réduite, l'acide dihydrolipoïque, la N-lipoyl-lysine ou encore l'acide rétinoïque,
et W représente
Glu-Gln-Arg, Arg-Lys, Arg-Lys-Asp, Arg, Glu-Gln, Gly-Gln-Gln ou une liaison,
et Z représente
Leu-DOPA, DOPA-NH2 ou HomoPhe-NH2,

Avantageusement, la séquence peptidique est conjuguée chimiquement ou physiquement avec les acides A. Les peptides conjugués selon l'invention sont liés sous forme de sels, d'esters ou d'amides à ces acides A, la fraction acide carboxilique de l'acide assurant la liaison.

Les acides aminés dans le peptide de formule (I) peuvent avoir une configuration D, L ou DL.

Autrement dit, les conjugués peptidiques de formule (I) peuvent comporter un ou plusieurs atomes de carbone asymétriques. Ils peuvent donc exister sous forme d'énantiomères ou de diasteréoisomères. Ces énantiomères, diastéréoisomères, ainsi que leurs mélanges, y compris les mélanges racémiques font partie de l'invention.

Les conjugués peptidiques de formule (II) sont des dérivés de faible poids moléculaire qui sont obtenus sous forme d'amides du composé de formule (III).

De plus, les peptides de formule (I) et les conjugués peptidiques de formule (II) peuvent être couplés avec du zinc, sous forme de sel pour former des complexes.

Dans le cadre de la présente invention, on entend par :
- Lys, la lysine,
- Asp, l'acide aspartique,
- Val, la valine,
- Arg, l'arginine,
- Tyr, la tyrosine,
- DOPA, la dihydroxyphénylalanine,
- HomoPhe, l'homophénylalanine.

Il est également précisé que les conjugués peptidiques mentionnés ci-dessus et faisant l'objet de la présente invention, peuvent être obtenus sous la forme terminale NH2 (autrement dit présentant une fonction amide) et sous la forme terminale OH (autrement dit présentant une fonction acide carboxylique).

De préférence, l'acide de formule (III) est un acide gras polyinsaturé, c'est-à-dire comportant de 1 à 6 insaturations. De manière encore plus préférée, il s'agit d'un acide oméga-3.

Parmi ces acides oméga-3 on peut notamment citer l'acide α-linolénique, l'acide cervonique, l'acide timnodonique et l'acide pinolénique. Les acides cervonique, timnodonique et pinolénique sont également connus sous les dénominations respectives d'acide 4 ,7,10, 13,16, 19-docosahexaenoïque (DHA), d'acide 5, 8, 11, 14, 17-eicosapentaénoïque (EPA) et d'acide 5, 9, 12-octodécatriénoïque.

Lorsque A représente un acide monocarboxylique de formule générale (III), il peut être avantageusement choisi parmi l'acide acétique, l'acide myristique, l'acide palmitique, les acides hydroxydécénoïques et décénoïques et notamment, l'acide trans-10-hydroxy-Δ2-décénoïque et l'acide trans-oxo-9-decene-2-oïque.

Parmi les conjugués peptidiques de l'invention on peut citer les conjugués peptidiques suivants :
1- A-Arg-Lys-Asp-Val-DHomoPhe-NH2
2- A-Arg-Lys-Asp-Val-HomoPhe-NH2 (SEQ ID N°5)
3- A-Lys-Asp-Val-DOPA-NH2 (SEQ ID N°6)
4- A-DLys-Asp-Val-DOPA-NH2
5- A-Arg-Lys-Asp-Val-DOPA-NH2 (SEQ ID N°7)

Les conjugués peptidiques pour lesquels A est choisi parmi l'acide lipoïque et l'acide acétique sont tout particulièrement adaptés, dans le cadre de la présente invention.

Les conjugués peptidiques objet de la présente invention, peuvent être obtenus soit par synthèse chimique classique, soit par synthèse enzymatique, selon des procédés quelconques connus de l'homme du métier.

Les peptides ou leurs conjugués peptidiques peuvent être administrés pour leur utilisation cosmétique par voie topique. Ils peuvent aussi être utilisés dans des compléments alimentaires, autrement dit dans le domaine nutraceutique par voie orale.

Les conjugués peptidiques selon l'invention sont préférentiellement administrés, par voie topique.

La présente invention a également pour objet un peptide ou un conjugué peptidique selon l'invention, à titre de médicament ainsi que l'utilisation d'un peptide ou d'un conjugué peptidique selon l'invention pour la préparation d'une composition destinée au traitement préventif et curatif de l'alopécie.

Selon un autre aspect, la présente invention a également pour objet une composition cosmétique ou dermatologique comprenant un peptide ou un conjugué peptidique selon la présente invention ou encore un complément alimentaire comprenant un peptide ou un conjugué peptidique selon la présente invention éventuellement en association avec un composé améliorant la repousse des cheveux tel que défini ci-après.

La composition cosmétique ou dermatologique peut avantageusement être appliquée sur l'ensemble du cuir chevelu.

La composition cosmétique ou dermatologique peut par exemple se présenter sous forme d'une lotion, d'un shampoing traitant, d'un spray, d'un gel ou d'une crème traitante.

Dans la composition cosmétique topique, le conjugué peptidique selon l'invention peut être présent à une concentration comprise entre 10⁻⁸ M et 10⁻³ M, de préférence comprise entre 10⁻⁷ M et 10⁻⁵ M.

Enfin, un autre objet de la présente invention concerne la méthode de traitement cosmétique pour lutter contre la chute des cheveux comprenant l'application sur le cuir chevelu d'une composition comprenant un peptide ou un conjugué peptidique de l'invention seul ou en association comme décrit ci-après ou encore comprenant l'administration par voie orale d'un complément alimentaire contenant une peptide ou un conjugué peptidique de l'invention, seul ou en association comme décrit ci-après.

Il est possible d'utiliser en association avec les conjugués peptidiques selon l'invention des composés améliorant encore l'activité sur la repousse et ayant déjà été décrits pour cette activité.

Parmi ces composés on peut citer :
- des peptides stimulant la production de collagène natif entraînant un renforcement de la matrice extracellulaire,
- le minoxidil,
- des esters de l'acide nicotinique,
- des agents anti-inflammatoires, plus particulièrement de peptides à activité anti-inflammatoire,
- l'acide rétinoique, ses dérivés et le rétinol,
- des inhibiteurs de la 5α -réductase.

Parmi les composés améliorant encore la repousse du cheveu et pouvant être associés au peptide ou au conjugué peptidique, on peut encore citer les peptides répondant à la formule générale (I)

X-Gly-His-Lys-Y (I)

(SEQ ID N° 8-9)
ou leurs conjugués répondant à la formule générale (II)

A-X-Gly-His-Lys-Y (II)

(SEQ ID N° 10-11)
dans lesquelles.
A est tel que défini ci-dessus,
X représente une chaîne de 1 à 3 résidus Lys, éventuellement méthylés ou lorsqu'il s'agit de la formule (II) une liaison,
Y représente un groupe -OH ou -NH2,
ou bien A-X représente un atome d'hydrogène,
sous forme d'énantiomères ou de diastéréoisomères, ainsi que leurs mélanges, y compris les mélanges racémiques et les complexes avec le zinc qui peuvent être formés avec ces peptides ou conjugués peptidiques.

Les compositions cosmétiques selon la présente invention, destinées à l'application topique sur le cuir chevelu, peuvent en outre comprendre un filtre UVB permettant la photoprotection du cuir chevelu. Ainsi, parmi les filtres UVB adaptés on peut citer, sous leur nom INCI:
- L'acide p-aminobenzoïque ou PABA et ses esters :
   *EthylhexyldimethylPABA
   *PEG-25PABA
- Les cinnamates :
   *Ethylhexyl Methoxycinnamate
   *Isoamyl p-Methoxycinnamate
   *Octocrylene
- les Salicylates :
   *Homosalate
   *Ethylhexyl Salicylate
- les benzimidazoles :
   *Phenylbenzimidazole Sulfonic Acid
- les dérivés Benzylidène camphres
   *4-Methylbenzylidene Camphor
   *Benzilidene Camphor
   *Camphor Benzalkonium Methosulfate
   *Polyacrylamidomethyl Benzylidene Camphor
- les triazines :
   *Ethylhexyl Triazone
   *Diethylhexyl Butamido Triazone.

Les peptides de l'invention ont fait l'objet d'essais pharmacologiques permettant de montrer leur activité anti-chute des cheveux.

### Effets des différents peptides sur la croissance de vibrisses de souris in vitro

Afin de montrer l'effet stimulateur des peptides dérivés de la thymopoïétine sur la croissance pilaire, des follicules pileux en phase anagène dé vibrisses de souris, sont mis en culture selon la technique décrite par Philpott (Philpott et col. 1994. Human Hair growth in vitro : a model for the study of hair biology. Journal of dermatological science 7 : S55-S72) en présence ou non de peptides dérivés de la thymopoïétine. La croissance de la tige pilaire de ces follicules pileux est suivie durant plusieurs jours (JO à J4). Les résultats sont reportés dans le tableau ci-après pour les peptides 1- et 2- décrits ci-dessus, pour lesquels A est l'acide acétique. Ces résultats montrent que ces peptides stimulent la croissance pilaire lorsque les follicules pileux sont maintenus en survie *in vitro.*

| | Témoin | Peptide 1-10⁻⁷M | Peptide 2-10⁻⁷M |
|---|---|---|---|
| J0 | 0, 00 | 0, 00 | 0, 00 |
| J1 | 0, 27 | 0, 90 | 0, 83 |
| J2 | 0, 43 | 1, 38 | 1, 46 |
| J3 | 0, 55 | 1, 86 | 1, 62 |
| J4 | 0, 55 | 1, 87 | 1, 62 |

Les exemples de formulation suivants illustrent la présente invention.

### Exemple 1 : lotion comprenant le conjugué peptidique Ac-Lys-Asp-Val-DOPA-NH2

| | (en g) |
|---|---|
| - Peptide Ac-Lys-Asp-Val-DOPA-NH2 | 5.10⁻⁶ |
| - Ethanol à 95° | 60 |
| - Propylène glycol | 10 |
| - Eau - conservateurs - parfum | qsp 100 |

### Exemple 2 : lotion comprenant le conjugué peptidique Ac-Arg-Lys-Asp-Val-HomoPhe-NH2

| | (en g) |
|---|---|
| - Peptide Ac-Arg-Lys-Asp-Val-HomoPhe-NH2 | 10⁻⁵ |
| - Eau | 81 |
| - Keltrol T | 0,5 |
| - Techpolymer MB-4C | 1 |
| - Sepigel 305 | 0,5 |
| - Huile de Silicone 0,2 1401 | 2 |
| - Butylène glycol | 5 |

## Revendications

1. Peptide répondant à la formule (I)
W-Lys-Asp-Val-Z (I)
ou son conjugué peptidique répondant à la formule (II)
A-W-Lys-Asp-Val-Z (II)
dans lesquelles
A représente le radical correspondant à
- un acide monocarboxylique de formule générale (III)
HOOC-R (III)
dans laquelle
R représente un radical aliphatique en C1-C24, linéaire ou ramifié, éventuellement substitué par un groupe hydroxy, pouvant comporter une ou plusieurs insaturations, avantageusement de 1 à 6 insaturations,
- l'acide lipoïque ou sa forme réduite, l'acide dihydrolipoïque, la N-lipoyl-lysine ou encore l'acide rétinoïque,
W représente
Glu-Gln-Arg, Arg-Lys, Arg-Lys-Asp, Arg, Glu-Gln, Gly-Gln-Gln ou une liaison,
et Z représente
Leu-DOPA, DOPA-NH2 ou
HomoPhe-NH2,
sous forme d'énantiomères ou de diasteréoisomères, ainsi que leurs mélanges, y compris les mélanges racémiques, le peptide de formule (I) et le conjugué peptidique de formule (II) pouvant se présenter sous forme de complexes avec le zinc.

2. Peptide ou conjugué peptidique selon la revendication 1, **caractérisé en ce que** l'acide de formule générale (III) est un acide oméga-3 choisi parmi l'acide α -linolénique, l'acide cervonique, l'acide timnodonique et l'acide pinolénique ou bien un radical aliphatique en C1-C24 choisi parmi l'acide acétique, l'acide myristique, l'acide palmitique, les acides hydroxydécénoïques et décénoïques et notamment, l'acide trans- 10-hydroxy-Δ2-décénoïque et l'acide trans-oxo-9-decene-2-oïque ou bien un acide choisi parmi l'acide lipoïque ou sa forme réduite, l'acide dihydrolipoïque, la N-lipoyl-lysine ou encore l'acide rétinoïque.

3. Peptide ou conjugué selon la revendication 1 ou 2, **caractérisé en ce que** A est choisi parmi l'acide lipoïque et l'acide acétique.

4. Conjugué peptidique selon l'une quelconque des revendications 1 à 3, **caractérisé en ce qu'**il est choisi parmi
1- A-Arg-Lys-Asp-Val-DHomoPhe-NH2
2- A-Arg-Lys-Asp-Val-HomoPhe-NH2 (SEQ ID N°5)
3- A-Lys-Asp-Val-DOPA-NH2 (SEQ ID N°6)
4- A-DLys-Asp-Val-DOPA-NH2
5- A-Arg-Lys-Asp-Val-DOPA-NH2 (SEQ ID N°7)
A étant un acide de formule générale (III) telle que définie dans l'une quelconque des revendications 1 à 3.

5. Composition cosmétique ou pharmaceutique **caractérisée en ce qu'**elle contient un peptide ou un conjugué selon l'une quelconque des revendications 1 à 4.

6. Composition cosmétique ou dermatologique selon la revendication 5, **caractérisée en ce qu'**elle est destinée à l'administration topique.

7. Peptide ou conjugué peptidique selon l'une quelconque des revendications 1 à 4 à titre de médicament.

8. Peptide ou conjugué peptidique selon l'une quelconque des revendications 1 à 4 à titre de médicament destiné au traitement préventif et curatif de l'alopécie.

9. Utilisation d'un peptide ou d'un conjugué peptidique selon l'une quelconque des revendications 1 à 4 pour la préparation d'une composition destinée au traitement préventif et curatif de l'alopécie.

10. Composition cosmétique destinée à lutter contre la chute des cheveux, **caractérisée en ce qu'**elle contient un peptide ou un conjugué peptidique selon l'une quelconque des revendications 1 à 4.

11. Composition cosmétique destinée à lutter contre la chute des cheveux selon la revendication 10, **caractérisée en ce qu'**elle contient en outre un composé améliorant la repousse des cheveux, choisi parmi le minoxidil, des esters de l'acide nicotinique, des agents anti-inflammatoires, de l'acide rétinoïque ou ses dérivés, du rétinol ou des inhibiteurs de la 5a-réductase.

12. Composition cosmétique destinée à lutter contre la chute des cheveux selon la revendication 10, **caractérisée en ce qu'**elle contient en outre un peptide répondant à la formule générale (I)
X-Gly-His-Lys-Y (I)
(SEQ ID N° 8-9)
ou son conjugué répondant à la formule générale (II)
A-X-Gly-His-Lys-Y (II) (SEQ ID N° 10-11)
dans lesquelles
A est tel que défini dans l'une quelconque des revendications 1 à 3,
X représente une chaîne de 1 à 3 résidus Lys, éventuellement méthylés ou lorsqu'il s'agit de la formule (II) une liaison,
Y représente un groupe -OH ou -NH2,
ou bien A-X représente un atome d'hydrogène,
sous forme d'énantiomères ou de diasteréoisomères, ainsi que leurs mélanges, y compris les mélanges racémiques et les complexes avec le zinc qui peuvent être formés avec ces peptides ou conjugués peptidiques.

13. Composition cosmétique destinée à lutter contre la chute des cheveux selon l'une quelconque des revendications 10 à 12, **caractérisée en ce qu'**elle contient un filtre UVB choisi parmi l'acide p-aminobenzoïque ou PABA et ses esters tels que l'EthylhexyldimethylPABA ou le PEG-25PABA ; les cinnamates tels que l'Ethylhexyl Methoxycinnamate, l'Isoamyl p-Methoxycinnamate, l'Octocrylene ; les Salicylates tels que l'Homosalate, l'Ethylhexyl Salicylate ; les benzimidazoles tels que le Phenylbenzimidazole Sulfonic Acid ; les dérivés benzylidène camphres tels que le 4-Methylbenzylidene Camphor, le Benzilidene Camphor, le Camphor Benzalkonium Methosulfate et le Polyacrylamidomethyl Benzylidene Camphor et les triazines telles que l'Ethylhexyl Triazone et le Diethylhexyl Butamido Triazone.

14. Complément alimentaire **caractérisé en ce qu'**il contient un peptide ou un conjugué peptidique selon l'une quelconque des revendications 1 à 4 ainsi qu'éventuellement un composé améliorant la repousse des cheveux tel que défini dans les revendications 11 ou 12.

## Claims

1. Peptide corresponding to the formula (I)
W-Lys-Asp-Val-Z (I)
or its peptide conjugate corresponding to the formula (II)
A-W-Lys-Asp-Val-Z (II)
in which
A represents the radical corresponding to
- a monocarboxylic acid of general formula (III)
HOOC-R (III)
in which
R represents a C1-C24 aliphatic radical, linear or branched, possibly substituted by a hydroxy group, which can include one or more unsaturations, advantageously from 1 to 6 unsaturations,
- lipoic acid or its reduced form, dihydrolipoic acid, N-lipoyl-lysine, or retinoic acid,
W represents
Glu-Gln-Arg, Arg-Lys, Arg-Lys-Asp, Arg, Glu-Gln, Gly-Gln-Gln or a chemical bond,
and Z represents
Leu-DOPA, DOPA-NH2 or
HomoPhe-NH2,
in the form of enantiomers or diastereomers, as well as their mixtures, including racemic mixtures, the peptide of formula (I) and the peptide conjugate of formula (II) which can be present in the form of complexes with zinc.

2. Peptide or peptide conjugate according to claim 1, wherein the acid of general formula (III) is an omega-3 acid chosen among α-linolenic acid, cervonic acid, timnodonic acid and pinolenic acid, or an aliphatic C1-C24 radical chosen among acetic acid, myristic acid, palmitic acid, hydroxydecenoic and decenoic acids, and in particular, trans-10-hydroxy-Δ2-decenoic acid and trans-oxo-9-decene-2-oic acid, or an acid chosen among lipoic acid or its reduced form, dihydrolipoic acid, N-lipoyl-lysine, or retinoic acid.

3. Peptide or conjugate according to claim 1 or claim 2, wherein A is selected among lipoic acid and acetic acid.

4. Peptide conjugate according to one of the claims 1 to 3, wherein it is selected among
1- A-Arg-Lys-Asp-Val-DHomoPhe-NH2
2- A-Arg-Lys-Asp-Val-HomoPhe-NH2 (SEQ ID NO. 5)
3- A-Lys-Asp-Val-DOPA-NH2 (SEQ ID NO. 6)
4- A-DLys-Asp-Val-DOPA-NH2
5- A-Arg-Lys-Asp-Val-DOPA-NH2 (SEQ ID NO. 7)
A being an acid of general formula (III) such as defined in one of the claims 1 to 3.

5. Cosmetic or pharmaceutical formulation wherein it contains a peptide or a conjugate according to one of the claims 1 to 4.

6. Cosmetic or dermatological formulation according to claim 5, wherein it is intended for topical application.

7. Peptide or peptide conjugate according to one of the claims 1 to 4 for use as a medicine.

8. Peptide or peptide conjugate according to one of the claims 1 to 4 for use as a medicine intended for the preventive and curative treatment of alopecia.

9. Use of a peptide or a peptide conjugate according to one of the claims 1 to 4 for the preparation of a formulation intended for the preventive and curative treatment of alopecia.

10. Cosmetic formulation intended to combat hair loss, wherein it contains a peptide or a peptide conjugate according to one of the claims 1 to 4.

11. Cosmetic formulation intended to combat hair loss according to claim 10, wherein it contains in addition a compound that improves hair growth, selected among minoxidil, nicotinic acid esters, anti-inflammatory agents, retinoic acid or its derivatives, retinol, or 5α-reductase inhibitors.

12. Cosmetic formulation intended to combat hair loss according to claim 10, wherein it contains in addition a peptide corresponding to the general formula (I)
X-Gly-His-Lys-Y (I)
(SEQ ID NO. 8-9)
or its conjugate corresponding to the general formula (II)
A-X-Gly-His-Lys-Y (II)
(SEQ ID NO. 10-11)
in which
A is such as defined in one of the claims 1 to 3,
X represents a chain of 1 to 3 Lys residues, possibly methylated or, in the case of the formula (II), a chemical bond,
Y represents an -OH or -NH2 group,
or A-X represents a hydrogen atom,
in the form of enantiomers or diastereomers, as well as their mixtures, including racemic mixtures and complexes with zinc which can be formed with these peptides or peptide conjugates.

13. Cosmetic formulation intended to combat hair loss according to one of the claims 10 to 12, wherein it contains a UVB filter chosen among P-aminobenzoic acid or PABA and its esters such as Ethylhexyl dimethyl PABA or PEG-25 PABA; the cinnamates such as Ethylhexyl methoxycinnamate, Isoamyl p-methoxycinnamate, Octocrylene; salicylates such as Homosalate, Ethylhexyl salicylate; benzimidazoles such as Phenylbenzimidazole sulfonic acid; benzylidene camphor derivatives such as 4-Methylbenzylidene camphor, Benzylidene camphor, Camphor benzalkonium methosulfate and Polyacrylamidomethyl benzylidene camphor and the triazines such as Ethylhexyl triazone and Diethylhexyl butamido triazone.

14. Food supplement wherein it contains a peptide or a peptide conjugate according to one of the claims 1 to 4, as well as, possibly, a compound that improves hair growth such as defined in claims 11 or 12.

## Patentansprüche

1. Peptid, welches der Formel (I) entspricht
W-Lys-Asp-Val-Z (I),
oder dessen Peptidkonjugat, welches der Formel (II) entspricht
A-W-Lys-Asp-Val-Z (II),
in welchen
A für den Rest, welcher
- einer Monocarbonsäure der allgemeinen Formel (III)
HOOC-R (III),
in welcher
R für einen aliphatischen C₁-C₂₄-Rest, linearen oder verzweigten, gegebenenfalls durch eine Hydroxygruppe substituierten, welcher eine oder mehrere
Ungesättigtheiten, in vorteilhafter Weise 1 bis 6 Ungesättigtheiten, umfassen kann, steht,
- Liponsäure oder deren reduzierter. Form, Dihydroliponsäure, N-Lipoyllysin oder ferner Retinsäure entspricht,
steht,
W für Glu-Gln-Arg, Arg-Lys, Arg-Lys-Asp, Arg, Glu-Gln, Gly-Gln-Gln oder eine Bindung steht
und Z für Leu-DOPA, DOPA-NH₂ oder HomoPhe-NH₂ steht,
in Form von Enantiomeren oder Diastereomeren wie auch deren Mischungen, einschließlich der racemischen Mischungen, wobei das Peptid der Formel (I) und das Peptidkonjugat der Formel (II) in Form von Komplexen mit Zink vorliegen können.

2. Peptid oder Peptidkonjugat nach Anspruch 1, **dadurch gekennzeichnet, dass** die Säure der allgemeinen Formel (III) eine Omega-3-Säure, ausgewählt unter α-Linolensäure, Cervonsäure, Timnodonsäure und Pinolensäure, oder ebenso ein aliphatischer C₁-C₂₄-Rest, ausgewählt unter Essigsäure, Myristinsäure, Palmitinsäure, den Hydroxydecen- und Decensäuren, und insbesondere trans-10-Hydroxy-Δ2-decensäure und trans-Oxo-9-decen-2-säure oder ebenso eine Säure, ausgewählt unter Liponsäure oder deren reduzierter Form, Dihydroliponsäure, N-Lipoyllysin oder ferner Retinsäure ist.

3. Peptid oder Konjugat nach Anspruch 1 oder 2, **dadurch gekennzeichnet, dass** A unter Liponsäure und Essigsäure ausgewählt wird.

4. Peptidkonjugat nach einem der Ansprüche 1 bis 3, **dadurch gekennzeichnet, dass** es ausgewählt wird unter:
1 - A-Arg-Lys-Asp-Val-DHomoPhe-NH₂
2 - A-Arg-Lys-Asp-Val-HomoPhe-NH₂ (SEQ ID NO. 5)
3 - A-Lys-Asp-Val-DOPA-NH₂ (SEQ ID NO. 6)
4 - A-DLys-Asp-Val-DOPA-NH₂
5 - A-Arg-Lys-Asp-Val-DOPA-NH₂ (SEQ ID NO. 7),
wobei A eine Säure der allgemeinen Formel (III), wie in einem der Ansprüche 1 bis 3 definiert, ist.

5. Kosmetische oder pharmazeutische Zusammensetzung, **dadurch gekennzeichnet, dass** sie ein Peptid oder ein Konjugat nach einem der Ansprüche 1 bis 4 enthält.

6. Kosmetische oder pharmazeutische Zusammensetzung nach Anspruch 5, **dadurch gekennzeichnet, dass** sie für eine topische Verabreichung bestimmt ist.

7. Peptid oder Peptidkonjugat nach einem der Ansprüche 1 bis 4 als Arzneimittel.

8. Peptid oder Peptidkonjugat nach einem der Ansprüche 1 bis 4 als Arzneimittel, welches für die präventive und kurative Behandlung von Alopezie bestimmt ist.

9. Verwendung eines Peptids oder eines Peptidkonjugats nach einem der Ansprüche 1 bis 4 für die Herstellung einer Zusammensetzung, die für die präventive und kurative Behandlung von Alopezie bestimmt ist.

10. Kosmetische Zusammensetzung, die dazu bestimmt ist, Haarausfall zu bekämpfen, **dadurch gekennzeichnet, dass** sie ein Peptid oder ein Peptidkonjugat nach einem der Ansprüche 1 bis 4 enthält.

11. Kosmetische Zusammensetzung, die dazu bestimmt ist, Haarausfall zu bekämpfen, nach Anspruch 10, **dadurch gekennzeichnet, dass** sie außerdem eine Verbindung, welche das Nachwachsen der Haare verbessert, ausgewählt unter Minoxidil, Nicotinsäureestern, entzündungshemmenden Mitteln, Retinsäure oder deren Derivaten, Retinol oder 5α-Reduktase-Inhibitoren, enthält.

12. Kosmetische Zusammensetzung, die dazu bestimmt ist, Haarausfall zu bekämpfen, nach Anspruch 10, **dadurch gekennzeichnet, dass** sie außerdem ein Peptid, welches der allgemeinen Formel (I) entspricht
X-Gly-His-Lys-Y (I)
(SEQ ID NO. 8-9),
oder dessen Konjugat, welches der allgemeinen Formel (II) entspricht
A-X-Gly-His-Lys-Y (II)
(SEQ ID NO. 10-11),
in welchen
A wie in einem der Ansprüche 1 bis 3 definiert ist,
X für eine Kette von 1 bis 3 Lys-Resten, die gegebenenfalls methyliert sind, oder, wenn es sich um die Formel (II) handelt, eine Bindung steht,
Y für eine Gruppe -OH oder -NH₂ steht,
oder alternativ A-X für ein Wasserstoffatom steht,
in Form von Enantiomeren oder Diastereomeren wie auch deren Mischungen, einschließlich der racemischen Mischungen und der Komplexe mit Zink, die mit diesen Peptiden oder Peptidkonjugaten gebildet werden können, enthält.

13. Kosmetische Zusammensetzung, die dazu bestimmt ist, Haarausfall zu bekämpfen, nach einem der Ansprüche 10 bis 12, **dadurch gekennzeichnet, dass** sie einen UVB-Filter, ausgewählt unter p-Aminobenzoesäure oder PABA und deren Estern, wie EthylhexyldimethylPABA oder PEG-25PABA; den Cinnamaten, wie Ethylhexylmethoxycinnamat, Isoamyl-p-methoxycinnamat, Octocrylen; den Salicylaten, wie Homosalat, Ethylhexylsalicylat; den Benzimidazolen, wie Phenylbenzimidazolsulfonsäure; den Benzylidencampher-Derivaten, wie 4-Methylbenzylidencampher, Benzylidencampher, Campherbenzalkoniummethosulfat und Polyacrylamidomethylbenzylidencampher, und den Triazinen, wie Ethylhexyltriazon und Diethylhexylbutamidotriazon, enthält.

14. Nahrungsergänzungsmittel, **dadurch gekennzeichnet, dass** es ein Peptid oder ein Peptidkonjugat nach einem der Ansprüche 1 bis 4 sowie gegebenenfalls eine Verbindung, welche das Nachwachsen der Haare verbessert, wie in den Ansprüchen 11 oder 12 definiert, enthält.
